# EUROPEAN PATENT SPECIFICATION

(11) **EP 0 674 647 B1**
(45) Date of publication and mention of the grant of the patent: **05.03.1997**
(21) Application number: 94912147.9
(22) Date of filing: 15.12.1993
(51) Int. Cl.: C07H 1/06, C07H 21/00, C08L 33/26

(54) **ANALYTICAL TECHNIQUE FOR OLIGONUCLEOTIDE ANALOGS**
ANALYTISCHES VERFAHREN FÜR OLIGONUCLEOTID-ANALOGA
TECHNIQUE ANALYTIQUE DESTINEE A DES ANALOGUES D'OLIGONUCLEOTIDES

(30) Priority: 16.12.1992 US 991466; 16.03.1993 US 32856
(43) Date of publication of application: 04.10.1995
(73) Proprietor: HYBRIDON, Inc., Worcester, Massachusetts 01605 (US)
(72) Inventor: COHEN, Aharon, S., Brookline, MA 02146 (US); VILENCHIK, Maria, Natick, MA 01760 (US)
(74) Representative: Vossius, Volker, Dr.
(86) International application number: PCT/US93/12372
(87) International publication number: WO 94/14824

(56) References cited:
- EP-A- 0 137 753
- EP-A- 0 497 448
- EP-A- 0 497 480
- US-A- 2 713 041
- US-A- 4 865 706
- ELECTROPHORESIS, vol.13, 1992 pages 484 - 486 M.J.ROCHELEAU ET. AL. 'Formamide modified polyacrylamide gels for DNA sequencing by capillary gel electrophoresis' cited in the application
- JOURNAL OF CHROMATOGRAPHY, vol.549, 1991, AMSTERDAM NL pages 446 - 451 A.WIDHALM ET. AL. 'Capillary zone electrophoresis with a linear, non-cross-linked polyacrylamide gel: separation of proteins according to molecular mass'
- NATURE, vol.292, 20 August 1981, LONDON GB pages 756 - 762 M.D.EDGE ET. AL. 'Total synthesis of a human leucocyte interferon gene' cited in the application
- JOURNAL OF CHROMATOGRAPHY, vol.516, 1990, AMSTERDAM NL pages 33 - 48 D.N.HEIGER ET. AL. 'Separation of DNA restriction fragments by high performance capillary electrophoresis with low and zero crosslinked polyacrylamide using continuous and pulsed electric fields' cited in the application
- PROCEEDINGS OF THE NATIONAL ACADEMY OF SCIENCES OF USA, vol.85, December 1988, WASHINGTON US pages 9660 - 9663 A.S.COHEN ET. AL. 'Rapid separation and purification of oligonucleotides by high-performance capillary gel electrophoresis' cited in the application

### SEQUENCE LISTING

(1) GENERAL INFORMATION:
   (i) APPLICANTS: Cohen, Aharon S. Vilenchik, Maria
   (ii) TITLE OF INVENTION: Analytic Technique for Oligonucleotide Analogs
   (iii) NUMBER OF SEQUENCES: 2
   (iv) CORRESPONDENCE ADDRESS:
      (A) ADDRESSEE: Allegretti & Witcoff, Ltd.
      (B) STREET: 75 State Street
      (C) CITY: Boston
      (D) STATE: Massachusetts
      (E) COUNTRY: U.S.A.
      (F) ZIP: 02109
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Kerner, Ann-Louise
      (B) REGISTRATION NUMBER: 33,523
      (C) REFERENCE/DOCKET NUMBER: 92614-A
   (ix) TELECOMMUNICATION INFORMATION:
      (A) TELEPHONE: 617/345-9100
      (B) TELEFAX: 617/345-9111
(2) INFORMATION FOR SEQ ID NO:1:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO:1:
   CGTATAGCCT GATGTCATAG CCGAT 25
(2) INFORMATION FOR SEQ ID NO:2:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (xi) SEQUENCE DESCRIPTION: SEQ ID NO:2:
   GACTCGAGGT CTGCTAACCT AGAT 24

## Description

This invention relates to the separation of mononucleotides and oligonucleotides. More particularly, this invention relates to the separation and characterization of modified and unmodified mononucleotides and oligonucleotides by high performance capillary electrophoresis.

Oligonucleotides that are complementary or "antisense" to specific genes or RNA sequences are relatively small, synthetic molecules having an average molecular weight of about 10 kilodaltons (kD). These antisense molecules have had widespread use in the field of selective gene regulation with consequent therapeutic implications. Phosphate backbone modification of such oligonucleotides provides nuclease resistance and greatly enhances the usefulness of these analogs. Such modifications include the substitution of phosphodiester internucleotide linkages with linkages such as methylphosphonates (Murakami et al. (1986) *Biochem* **24**:4041-4046; Agrawal et al. (1987) *Tetrahedron Lett.* **28**:3539-3542; Sarin et al. (1988) *Proc*. *Natl. Acad*. *Sci*. (USA) **85**:7448-7451), phosphorothioates (Burgers et al. *Biochemistry* **18**:592-596; Agrawal et al. (1988) *Proc*. *Natl. Acad. Sci*. (USA) **85**:7079-7083; Agrawal et al. (1989) *Nucleosides and Nucleotides* **8**:819-823; Agrawal et al. (1989) *Proc*. *Natl*. *Acad*. *Sci*. (USA) **86**:7790-7794), and phosphoramidates (Agrawal et al. (1988) *Proc. Natl. Acad. Sci*. (USA) **85**:7079-7083; Agrawal et al. (1989) *Nucleosides and Nucleotides* **8**:819-823).

Of special interest are phosphorothioate analogs in which one non-bridging oxygen atom has been substituted for a sulfur atom on the phosphate group in each internucleotide phosphate linkage. This modification is a conservative substitution which increases nuclease resistance without significantly impairing the hybridization of the antisense molecule with target mRNA. As synthesized, these modified oligonucleotides or analogs are usually found as diastereomeric mixtures due to chirality at their phosphorous group. In a context of new drug research, development and manufacturing of such analogs requires that the issues of oligomer length, base composition, base sequence, chemical purity, and stereochemical purity be successfully addressed.

Synthetic oligonucleotides are presently used in most laboratories using molecular biology techniques. As synthesized, these oligonucleotides generally exist as mixtures of truncated oligonucleotides in addition to the desired oligonucleotide. Since the purity and chemical identity of a particular oligonucleotide is crucial to many applications, the ability to characterize and separate synthetic oligonucleotides analogs on a routine basis is important.

The absolute length and the degree of length heterogeneity of prepared oligonucleotides have been assessed by electrophoresis in high resolution denaturing polyacrylamide slab gels (PAGE) *(see, e.g., Current Protocols in Molecular Biology,* Green Publishing and Wiley Interscience, N.Y., 1988) and by capillary gel electrophoresis through cross-linked polyacrylamide (6% T, 5% C) gels (Hjerten (1967) *Chromatogr. Rev.* **9**:122-213) containing from 10% to less than 30% (vol.:vol.) formamide (Rocheleau et al. (1992) *Electrophoresis* **13**:484-486). Detection of oligonucleotides separated on such gels has been accomplished by autoradiography and laser-induced fluorescence. These methods have not proven suitable for separating modified oligonucleotides. Furthermore, some of these gels, once used, are not easily removable from the capillary. To remedy this problem, gels containing up to 5% acrylamide monomer have been polymerized before filling the capillary (EPO 497 480). Ultrathin slab gels (less than 100 µm in thickness) have also been used for high speed DNA sequencing (Brumley et al. (1991) *Nucleic Acids Res*. **19**:4121-4126; Ansorge et al. (1990) *Nucleic Acids Res*. **18**:3419-3420). Alternative separation methods include ion exchange chromatography, reversed phase high pressure liquid chromatography (HPLC), and gel high performance capillary electrophoresis (HPCE) *(see, e.g.,* Edge et al. (1981) *Nature* **292**:756-762; U.S. Patent No. 4,865,707).

Oligonucleotides with phosphorothioate linkages are more difficult to resolve than phosphodiester-linked DNA due to the existence of diastereomer isomers (2ⁿ, where n = the number of chiral centers, which is equivalent to the number of phosphate groups). In addition, difficulty in resolution may be due to increased hydrophobicity of the former. These molecules, when separated, interact hydrophobically with ion exchange column supports and in many cases co-elute. Thus, they cannot be separated by the above methods in their existing formats.

The separation of phosphorothioate oligonucleotide analogs is problematic for other reasons as well. When phosphorothioate oligonucleotides are assembled using either methoxyphosphoramidite or H-phosphate chemistry, they are in the form of diastereomeric mixtures due to chirality at their phosphorous groups. As a result, although they migrate through polyacrylamide gels and HPLC columns like their corresponding phosphodiester counterparts, phosphorothioate oligonucleotides give broader peaks and run more slowly than phosphodiesters because of their increased hydrophobicity. They are also known to interact with the HPLC column support. In addition, phosphorothioates run into stereochemical problems when separated by reversed phase HPLC. General analytical methods have not been devised for establishing the ratio of the optical isomers at each unsymmetrical substitution phosphorous linkage in an analog having many such sites of local chirality.

HPLC of oligodeoxyribonucleotides containing one or two phosphorothioate internucleotide linkages using a reversed-phase column (RP-HPLC) has been reported (Stec et al. (1985) *J. Chromatogr.* **326**:263-280; Agrawal et al. (1990) *Nucleic Acids Res.* **18**:5419-5423). However, this method is of limited use because of the small differences in the hydrophobicity of these analogs with increasing chain length (Agrawal et al. (1990) *J. Chromatogr.* **509**:396-399).

Separation of oligodeoxyribonucleotide phosphorothioates containing 10 or fewer nucleotides has also been achieved by HPLC on strong anion-exchange (SAX) columns (Agrawal et al. (1990) *J. Chromatogr.* **509**:396-399). In this method, oligonucleotide phosphorothioates were converted to their phosphodiester counterparts in one step, and then were analyzed by HPLC. Unfortunately, oligonucleotides phosphorothioates containing more than 10 nucleotides can not be analyzed by this method because of their strong interaction with the SAX medium. Thus the separation of oligonucleotide phosphorothhioates by this method is limited by its oligonucleotide length dependency.

Length-dependent separation of phosphorothioate analogs by HPLC using a weak anion-exchange (WAX) column has also been accomplished (Meletev et al. (1992) *Analyt. Biochem.* **200**:342-346). However, the peaks obtained were broader than those obtained for their phosphodiester counterparts, possibly because of their diastereomeric backbone. Ion-pair HPLC has also been used to analyze oligonucleotide phosphorothioates (Bigelow et al. (1990) *J. Chromatogr*. **533**:131-140), but length-dependent separation was not achieved.

Thus, one technical problem underlying the present invention is to provide methods of separating unmodified and modified mononucleotides and oligonucleotides cleanly, rapidly, efficiently, and which are not limited by the size range or modification of the molecules being analyzed.

Another technical problem is to provide a novel substrate used for the separation of unmodified and modified mononucleotides and oligonucleotides which can be quickly and easily removed and replaced.

### SUMMARY OF THE INVENTION

A novel separation substrate and method of its use have been developed for the separation and characterization of unmodified and modified mononucleotides and oligonucleotides differing by as little as a single base. An advantage to this method is the relative ease by which samples of less than 1 nanogram in microliter or smaller volumes can be conveniently handled with on-line UV detection. Another advantage is the relative ease with which the separation substrate can be replaced with new substrate after use. Relative to slab gel and on-line UV regular gel high performance capillary electrophoresis (HPCE) operation, this new formulation can be very useful for process analysis as well as for purity assessment of antisense nucleotides in the pharmaceutical industry.

As used herein, a "mononucleotide analog" or "modified mononucleotide" is a base, including purines and pyrimidines, or modifications thereof, attached to the 1' end of the deoxyribose or ribose sugar, or modifications thereof, which is attached at its 5' position to a phosphate group. A "5'-substituted mononucleotide analog" includes a deoxyribose or ribose sugar, which is attached at its 5' position to a chemical group other than the phosphate group found in native nucleotides. Preferable chemical groups include alkyl phosphonates, phosphorothioates, alkyl phosphorothioates, phosphoramidates, phosphorodithioates, phosphate diesters, and phosphate triesters.

A "3'-substituted mononucleotide analog" includes a deoxyribose or ribose sugar attached at its 3' position to a chemical group other than the hydrogen found in native nucleotides.

"Modified oligonucleotides" or "oligonucleotide analog", as used herein, is a molecule containing at least two ribonucleotides or deoxyribonucleotides which are covalently linked via at least one synthetic linkage. A "synthetic internucleotide linkage" is a linkage other than a phosphodiester linkage between the 5' end of one nucleotide and the 3' end of another nucleotide in which the 5' internucleotide phosphate has been replaced with any number of chemical groups. Preferable synthetic linkages include alkyl phosphonates, phosphorothioates, alkyl phosphorothioates, phosphoramidates, phosphorodithioates, phosphatediesters and phosphate triesters. A "3',5'-substituted" mononucleotide or oligonucleotide is a modified mononucleotide or oligonucleotide having a sugar which, at both its 3' and 5' positions is attached to a chemical group other than a hydroxyl group (at its 3' position) and other than a phosphate group (at its 5' position). A modified oligonucleotide may also be capped.

The separation substrate used comprises at least 12% polymer that is no more than 1% cross-linked in at least 50% (volume:volume) organic solvent, the organic solvent being a denaturing agent. The preferred polymer is polyacrylamide, methyl cellulose and derivatives thereof, and polyvinyl alcohol. In one preferred aspect, the substrate of the invention includes at least 12% T polymerized acrylamide (or polyacrylamide) in at least 50% and preferably 60% (volume:volume) organic solvent. The term "T" refers to the percent of monomers (mass:volume). In one aspect of the invention, the substrate is a linear polyacrylamide. In some embodiments, the substrate contains about 12% to 20% T polyacrylamide, with from about 13% to 18% T being optimal. A linear gradient of about 13% to 18% T polyacrylamide is present in some aspects of the invention. The polyacrylamide may be non-cross-linked in some aspects of the invention. In other aspects the polyacrylamide may contain up to 1% cross-linking.

Useful denaturants include formamide, urea, and sodium dodecyl sulfate. A preferred denaturant is formamide present at a concentration of at least 60% (volume:volume). In some embodiments, the invention includes a substrate containing 18% T polyacrylamide in formamide, and may further include urea.

This invention also provides a method of separating unmodified and modified mononucleotides and oligonucleotides using the above-described substrate. The method includes contacting the substrate, which is in a high performance capillary, with the mononucleotide and/or oligonucleotides to be separated. An electric field greater than 200 volts/centimeter is applied across the substrate in the capillary, and the separated mononucleotides and/or oligonucleotides are detected. In preferred embodiments of the invention, an electric field of about 400 volts/cm is applied across the substrate.

In another aspect of the invention the substrate is removed from the capillary in an additional step (e) after the detection step (d). Removal is preferably accomplished by applying at least 2,11 kg/cm² (30 psi) pressure on one end of the capillary. Such pressure can be exerted manually or by means cf an automated system.

Molecules capable of being separated by this method include unmodified mononucleotides and oligonucleotides and mononucleotide and oligonucleotide analogs having from 1 to 50 bases. This method is also useful for separating such mononucleotides and oligonucleotide analogs having from 1 to 150, and even up to about 300 bases.

### BRIEF DESCRIPTION OF THE DRAWINGS

The foregoing and other objects of the present invention, the various features thereof, as well as the invention itself may be more fully understood from the following description, when read together with the accompanying drawings in which:
FIG. 1A is an electropherogram demonstrating the electrophoretic separation by high performance capillary electrophoresis of phosphorothioate failure sequences ranging in length from 1 to 50 bases in length;
FIG. 1B is an electropherogram demonstrating the electrophoretic separation by high performance capillary electrophoresis of phosphorothioate failure sequences ranging in length from 1 to 75 bases in length;
FIG. 2 is a calibration plot of migration time of the analogs separated in FIG. 1 versus base length in the analog; and
FIG. 3 is an electropherogram demonstrating the electrophoretic separation of a mixture of the 25mer (SEQ ID NO:1), the 24mer (SEQ ID NO:2), and failure sequences resulting from the syntheses of these phosphorothioate oligonucleotide analogs. Peak A represents the putative 23mer failure sequence from 24mer (SEQ ID NO:2) synthesis; peak B represents the 24mer (SEQ ID NO:2); peak C represents the putative 24mer failure sequence from 25mer (SEQ ID NO:1) synthesis; peak D represents the 25mer (SEQ ID NO:1); and peak E is unknown.

### DETAILED DESCRIPTION OF THE PREFERRED EMBODIMENTS

This invention provides a novel substrate and methods of using that substrate to separate unmodified and modified mononucleotides and oligonucleotides which may differ by only one base.

HPCE utilizing the novel substrate of the invention holds a unique position in the field of oligodeoxynucleotide separation due to its resolution power, ability to determine purity, speed, and automation. Because of the low current generated (µA) from the narrow bore columns (25 to 200 µm, inner diameter), high electric fields (hundreds of volts/cm) without excess Joule heating can be employed, resulting in very rapid, high resolution separations. As an instrument technique, HPCE is highly reproducible, is amenable to automation, and is thus a powerful alternative tool for antisense analysis. Furthermore, the substrate is unique because it is easily removable and replaceable.

Traditional capillary electrophoresis suggests the application of electric fields lower than 200 V/cm with low ionic strength buffer (not higher than 0.1 M Tris-borate-EDTA (TBE)) and low gel concentration in aqueous media for the separation of oligonucleotides. However, it has been discovered that the use of 0.2 M TBE buffer and an electric field of at least 200 V/cm gives very high resolution in certain gel substrates for the separation of oligonucleotide analogs.

The substrate used is a polymer such as polyacrylamide, methyl cellulose, polyvinyl alcohol, or derivatives thereof. The polymer may be up to 1% cross-linked but need not be cross-linked at all. It is important that the concentration of polymer in the capillary be 12% or higher to achieve this kind of resolution and efficiency. No gradient of polymer is required, but linear gradients of, for example, from about 12% to 20%, or more preferably, from about 13% to 18% polymer may be used.

The polymer must be suspended in at least 50% (volume:volume) organic solvent. Useful organic solvents are also denaturing agents which keep the oligonucleotides from assuming secondary structure, an obstacle to clean separation. Such denaturants include formamide, urea, and sodium dodecyl sulfate, among others. One particularly useful denaturant is formamide. To improve denaturation even more, a high concentration of urea (7 to 8.3 M) may also be added.

In the case of acrylamide, polymerization may be achieved by adding ammonium persulfate as a free radical catalyst and N,N,N',N'-tetramethylethylenediamine (TEMED) to the acrylamide solution. The substrate solution is then placed into the capillary where it polymerizes. A useful capillary is a microcapillary column (25 to 200 µm inner diameter) made of fused silicon, as described in U.S. Patent nos. 4,865,706 and 5,112,460.

The molecules which can be successfully separated on this substrate include unmodified mononucleotides and oligonucleotides and modified mononucleotides and oligonucleotides (analogs) such as 3'-, 5'-, and 3', 5' -substituted mononucleotides and oligonucleotides, and mononucleotides and oligonucleotide analogs having at least one phosphate group replaced with a chemical group such as a phosphorothioate, phosphorodithioate, phosphonate, alkyl phosphonate, alkyl phosphorothioate, phosphate ester, phosphate diester, phosphate triester, phosphoramidite, and phophoramidate, among others. An oligonucleotide analog can have at least one artificial or synthetic (i.e., non-phosphodiester) internucleotide linkage formed by such a chemical group. The preparation of these molecules is well known in the art (reviewed in Agrawal et al. *(Trends in Biotechnol*. (1992) **10**:152-158). For example, monomeric and oligomeric phosphorothioate analogs can be prepared using methods well known in the field such as methoxyphosphoramidite *(see, e.g.,* Agrawal et al. (1988) *Proc*. *Natl. Acad. Sci*. (USA) **85**:7079-7083) or H-phosphorate *(see, e.g.,* Froehler (1986) *Tetrahedron Lett.* **27**:5575-5578) chemistry. The synthetic methods described in Bergot et al. (*J. Chromatog. (1992)* **559**:35-47) can also be used. The products of these syntheses may include failure sequences as well as the desired oligonucleotide sequence. The failure sequences have at least one less base than the desired oligonucleotide, but the position of the missing base is unknown without subsequent sequencing analysis.

In order to separate the failure sequences from the desired oligonucleotides so produced, or in order to distinguish, characterize, and isolate different desired mononucleotides and/or oligonucleotide species from each other, the molecules to be examined are analyzed by HPCE using a capillary electrophoresis apparatus. Such an instrument is well known in the field (see, e.g., Cohen et al. (1988) *Proc. Natl. Acad. Sci.* (USA) **85**:9660-9663). The molecules to be separated are injected into the capillary by siphoning (in the case of open tube applications). Alternatively, the sample may be electrophoretically injected into the column by dipping the cathodic end of the capillary into the sample solution and applying a field of 400 v/cm for 1 to 3 sec. The sample is then run through the gel, and the separated analogs detected by UV, infrared, fluorescence, laser-induced fluorescence or other external monitoring methods.

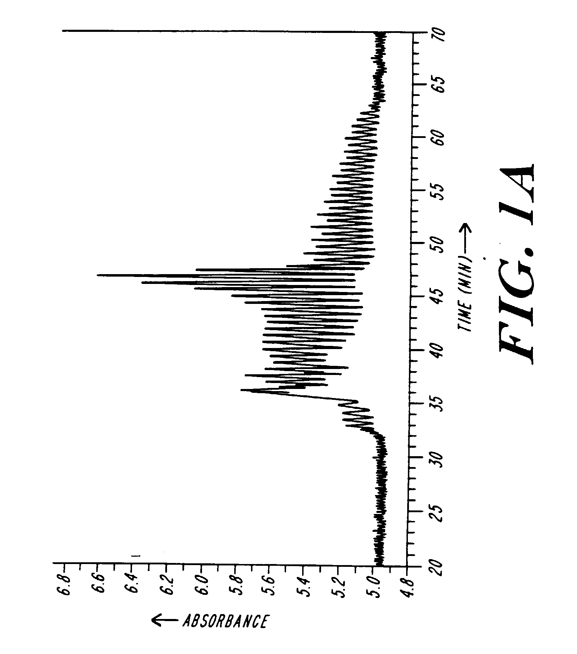

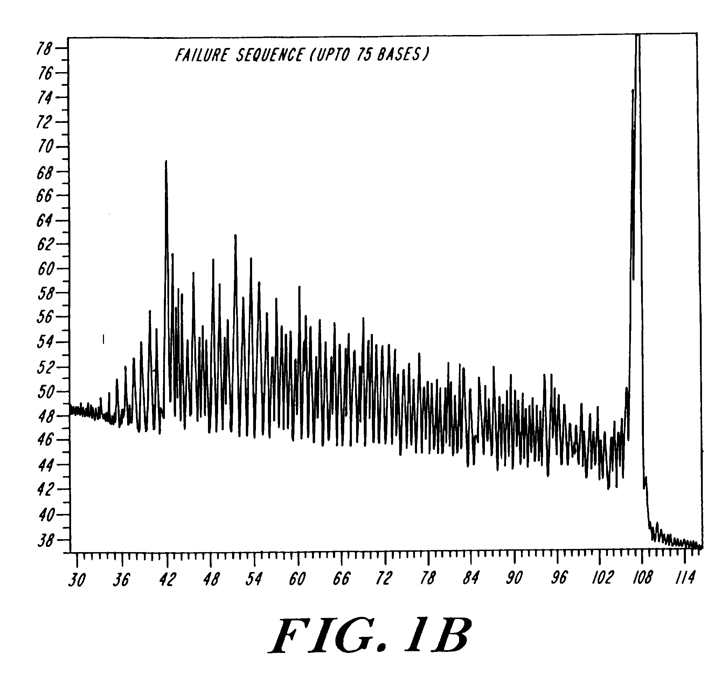

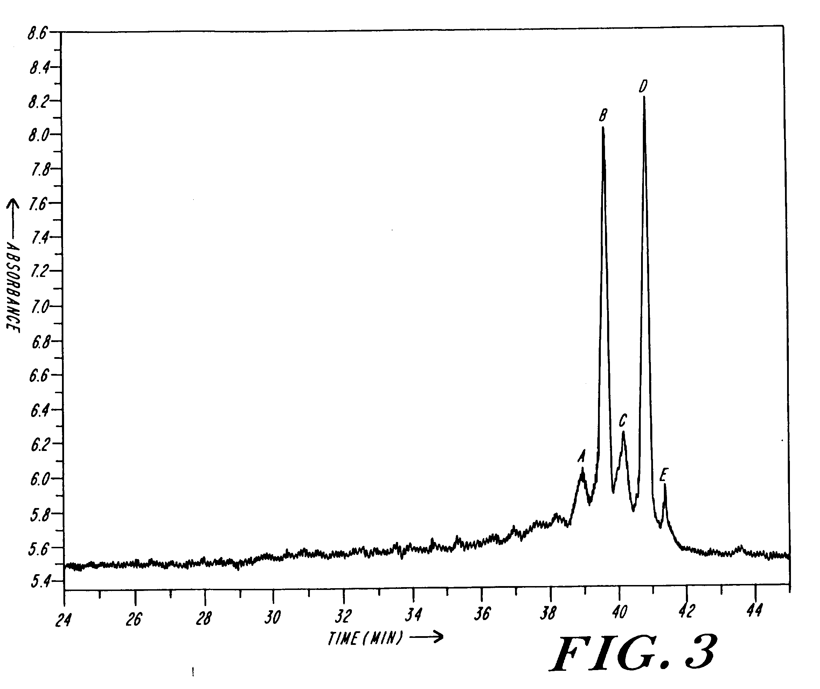

As demonstrated by the electropherograms in FIGS. 1A and 1B, this method enables the separation of oligonucleotide analogs differing in length by only one base. Furthermore, this method separates oligonucleotide analogs having the same length but differing in base sequence, as shown in Figure 3.

The used substrate, including polyacrylamide of 6% or higher T in formamide, can be easily removed from the capillary and replaced with fresh substrate, if desired. This is because the high formamide concentration in the substrate decreases its viscosity. Removal is accomplished using low pressure 2,11-3,52 kg/cm² (about 30 to 50 psi) applied manually or with the aid of an automated system. For example, a syringe can be used to apply the pressure required to push the substrate out of the capillary.

The following examples illustrate the preferred mode of making and practicing the present invention, but are not meant to limit the scope of the invention since alternative methods may be utilized to obtain similar results.

### EXAMPLES

### 1. HPCE Apparatus

The high performance capillary electrophoresis apparatus with UV detection and the preparation of substrate-filled capillary for the separation of DNA molecules are essentially the same as described in Cohen et al. (*Proc. Natl. Acad. Sci*. (USA) (1988) **85**:9660-9663) and Heiger et al. (*J. Chromatogr.* (1990) **516**:33-48). A 30 kV, 500 µA direct current high voltage power supply (Model ER/DM; Glassman, Whitehouse Station, NJ) was used to generate the potential across the capillary.

### 2. Preparation of Substrate-Filled Capillaries

Fused-silica capillary tubing (Polymicro Technologies, Phoenix, AZ) with inner diameter cf 75 µm, outer diameter of 375 µm, effective length of 20 cm, and total length of 30 cm, was treated with (methylacryloxypropyl)trimethoxysilane (Petrach Systems, Bristol, PA) and then filled with a carefully degassed 13 to 18% T linear polyacrylamide in aqueous or formamide solution in 0.2 M TBE buffer (0.2 M Tris borate, 4 mM EDTA), pH 8.3, with 7 to 8.3 M urea. Alternatively, capillaries were filled with a degassed solution of 13% or 18% T linear polyacrylamide. Polymerization was achieved by adding ammonium persulfate solution and TEMED. To remove impurities from the polyacrylamide, the capillary column was pre-electrolyzed at 6 kV for 30 to 60 minutes. During electrophoresis, the capillary was maintained at room temperature. Ultra-pure Trizma base, urea, acrylamide, and EDTA were purchased from Schwartz/Mann Biotech (Cleveland, OH). TEMED and ammonium persulfate were purchased from Bio-Rad (Richmond, CA).

### 3. Preparation of Oligonucleotides

The oligonucleotide phosphorothioate 25mer 5'-CGTATAGCCTGATGTCATAGCCGAT-3' (SEQ ID NO:1), 24-mer 5'-GACTCGAGGTCTGCTAACCTAGAT-3' (SEQ ID NO:2), and the failure sequences from the syntheses of various oligomers having a length of up to 50 bases and up to 150 bases (base sequences unknown) were synthesized using the procedure of Beaucage et al. (U.S. Patent No. 5,003,097), herein incorporated by reference. Briefly, oligodeoxyribonucleotides were synthesized on an automated synthesizer (Model 8700, Milligen/Biosearch, Bedford, MA) . Both normal phosphodiester oligodeoxyribonucleotides and their phosphorothioate analogues were assembled using H-phosphonate chemistry (Andrus et al. (1988) *Tetrahedron Lett.* **29**:61; Gregg et al. (1987) *Tetrahedron Lett.* **27**:4051). Synthesis was carried out on a 10-µmol scale, and after the chain elongation cycles the controlled pore glass support-bound oligonucleoside H-phosphonate was treated with 0.2 M sulfur in carbon disulfide:pyridine:triethylamine (12:12:1, volume:volume) to generate phosphorothioate internucleotide linkages. Deprotection of oligodeoxyribonucleotide was carried out with concentrated ammonia at 55°C for 8 hours. Deprotected oligodeoxyribonucleotides were then resuspended in distilled water.

### 4. Separation of Oligonucleotides

Samples were electrophoretically injected into the column by dipping the cathodic end of the capillary into the sample solution and applying a voltage of 400 V/cm for 2 seconds. Separation was achieved at a typical applied field of 400 V/cm. Each column was used for multiple injections. Periodically, a short section of the capillary at the injection end was trimmed.

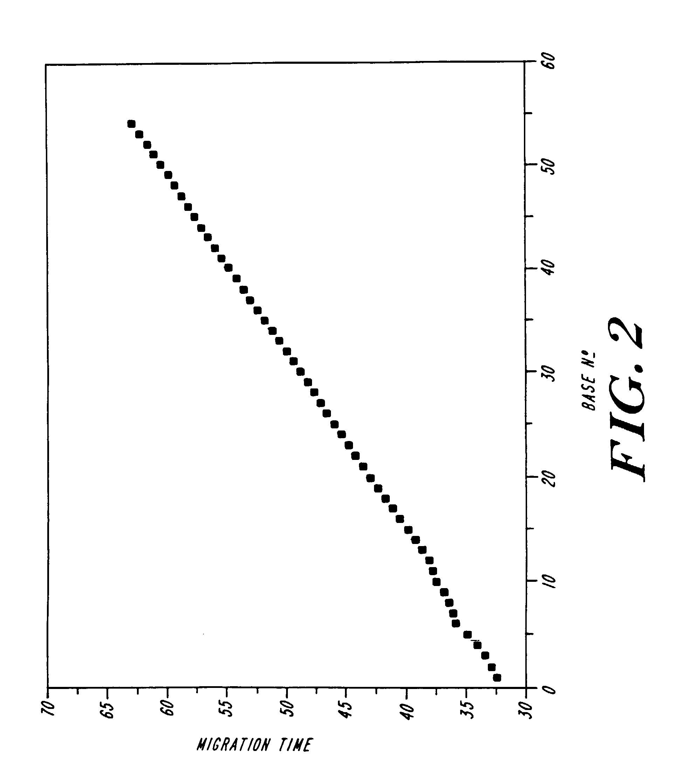

The failure sequence sample (containing oligonucleotides varying in length from 1 to 50 bases, from 1 to 75 bases, and from 1 to 150 bases) was suspended in water with final concentration 500 ng/ml. Each of these samples were separated on a capillary containing 15% T linear polyacrylamide. The column was developed with 60% formamide, 0.2 M TBE buffer, 8.3 M urea, pH 8.3. Electrophoresis was conducted under an applied electric field of 400 volts/cm and a current of 12 µA over a 20 cm migration distance. The results from the 1 to 50 and 1 to 75 base samples are shown in FIGS. 1A and 1B. When migration time was examined with respect to fragment length, a linear relationship (r²=0.9999) was observed (FIG. 2). This linear behavior of the phosphorothioate analogs is indicative of the lack of peak compression, and of migration according to molecular weight or size, each being important elements of successful oligonucleotide separation.

A sample containing a mixture of the 24mer (SEQ ID NO:2) and the 25mer (SEQ ID NO:1) phosphorothioate analogs (having different sequences but the same length) was suspended in water to final concentration 400 ng/ml. The sample was run on a capillary containing 13% T, 0% C, 7 M urea, 0.2 M TBE, pH 8.3. (The term "c" refers to a fraction: the amount of crosslinked polymer over the total monomer and cross-linked monomer). Electrophoresis was conducted under an electric field of 400 volts/cm and a current of 12 µA over a 20 cm migration distance. The results are shown in FIG. 3. The time window between elution of the 24mer and elution of the 25mer is large enough to accommodate an additional peak. This peak is presumed to be a failure sequence of the synthesized 25mer and is therefore a 24mer since this peak is migrating directly after the 25mer under denaturing conditions. The two 24mers are separated due to the difference in their base sequences.

### 5. Detection Method

Oligonucleotides were monitored by UV detection at wavelength 270 nm using a Spectra-100 spectrophotometer (Spectra Physics, San Jose, CA). The data were stored on an Ace IBM compatible PC computer via an analog to digital (A/D) converter (Model 970, Nelson Analytical, Cupertino, CA).

### 6. Removal and Replenishment of the Substrate

After detection of oligonucleotides in the substrate, the end of the capillary is contacted with the distal end of a series "C" Pressure Lok syringe (Rainin Instrument Co., Woburn, MA) with its plunger drawn out. Pressure is applied to the substrate in the capillary by gently pushing in the plunger of the syringe. Only about 30 to 50 psi pressure is required to quickly remove the used substrate from the capillary. The substrate is dispelled into a container and regenerated or disposed of. The capillary can then be refilled with fresh substrate.

Those skilled in the art will recognize, or be able to ascertain, using no more than routine experimentation, numerous equivalents to the specific substances and procedures described herein. Such equivalents are considered to be within the scope of this invention, and are covered by the following claims.

## Claims

1. A method of separating unmodified and modified mononucleotides and oligonucleotides comprising the steps of:
(a) providing a separation substrate in a high performance capillary, the separation substrate comprising at least 12% polymer that is no more than 1% cross-linked in at least 50% (volume:volume) organic solvent, the organic solvent being a denaturing agent;
(b) contacting the separation substrate with the mononucleotides and oligonucleotides to be separated;
(c) applying an electric field greater than 200 volts/cm across the separation substrate in the capillary; and
(d) detecting the separated mononucleotides and/or oligonucleotides.

2. The method of claim 1 wherein in step (a) a separation substrate is used which comprises a polymer selected from the group consisting of polyacrylamide, methylcellulose, and polyvinyl alcohol.

3. The method of claim 1 wherein in step (a) a separation substrate is used which contains from about 12% to 20% T polyacrylamide.

4. The method of claim 3 wherein in step (a) a separation substrate is used which contains a linear gradient of from about 13% to 18% T polyacrylamide.

5. The method of any one of claims 1 to 4 wherein in step (a) a denaturant selected from the group consisting of formamide, sodium dodecyl sulfate, and urea is used.

6. The method of claim 5 wherein in step (a) a separation substrate is used which contains formamide and optionally urea.

7. The method of any one of claims 1 to 6 wherein in step (c) an electric field of about 400 volts/cm across the separation substrate in the capillary is applied.

8. The method of claim 1 further comprising the additional step (e) of removing the separation substrate from the capillary after step (d).

9. The method of claim 8 wherein in step (e) a pressure of at least about 2,11 kg/cm² (about 30 pounds per square inch) is applied to an end of the capillary.

10. A separation substrate for separating unmodified and modified mononucleotides and oligonucleotides, comprising at least 12% polymer that is no more than 1% cross-linked in at least 50% (volume:volume) organic solvent, the organic solvent being a denaturing agent.

11. The separation substrate of claim 10 wherein the polymer is selected from the group consisting of polyacrylamide, methylcellulose, and polyvinyl alcohol.

12. The separation substrate of claim 11 comprising a linear polyacrylamide.

13. The separation substrate of claim 12 comprising about 12 to 20% T polyacrylamide.

14. The separation substrate of claim 10 wherein the denaturant is selected from the group consisting of formamide, urea, and sodium dodecyl sulfate.

15. The separation substrate of claim 14 wherein the denaturant is formamide.

16. The separation substrate of claim 15 comprising about 60% formamide.

17. The separation substrate of claim 15 or 16 further comprising urea.

## Patentansprüche

1. Verfahren zur Trennung unmodifizierter und modifizierter Mononucleotide und Oligonucleotide, umfassend die Schritte:
(a) Bereitstellung eines Trennsubstrates in einem Hochleistungskapillargefäß, das Trennsubstrat umfaßt mindestens 12% Polymer, das nicht mehr als 1% quervernetzt ist, in mindestens 50% organischem Lösungsmittel (Volumen:Volumen), wobei das organische Lösungsmittel ein denaturierendes Mittel ist;
(b) In-Kontakt-Bringen des Trennsubstrates mit den zu trennenden Mononucleotiden und Oligonucleotiden;
(c) Anlegen eines elektrischen Feldes größer als 200 Volt/cm über das Trennsubstrat in dem Kapillargefäß und
(d) Nachweis der getrennten Mononucleotide und/oder Oligonucleotide.

2. Verfahren nach Anspruch 1, worin in Schritt (a) ein Trennsubstrat verwendet wird, das ein Polymer umfaßt, ausgewählt aus der Gruppe bestehend aus Polyacrylamid, Methylzellulose und Polyvinylalkohol.

3. Verfahren nach Anspruch 1, worin in Schritt (a) ein Trennsubstrat verwendet wird, das etwa 12% bis 20% T-Polyacrylamid enthält.

4. Verfahren nach Anspruch 3, worin in Schritt (a) ein Trennsubstrat verwendet wird, das einen linearen Gradienten von etwa 13% bis 18% Polyacrylamid enthält.

5. Verfahren nach einem der Ansprüche 1 bis 4, worin in Schritt (a) ein Denaturierungsmittel, ausgewählt aus der Gruppe bestehend aus Formamid, Natriumdodecylsulfat und Harnstoff, verwendet wird.

6. Verfahren nach Anspruch 5, worin in Schritt (a) ein Trennsubstrat verwendet wird, das Formamid und gegebenenfalls Harnstoff enthält.

7. Verfahren nach einem der Ansprüche 1 bis 6, worin in Schritt (c) ein elektrisches Feld von etwa 400 Volt/cm über das Trennsubstrat in dem Kapillargefäß angelegt wird.

8. Verfahren nach Anspruch 1, ferner umfassend den zusätzlichen Schritt (e) des Entfernens des Trennsubstrates von dem Kapillargefäß nach Schritt (d).

9. Verfahren nach Anspruch 8, worin in Schritt (e) ein Druck von mindestens etwa 2,11 kg/cm² (etwa 30 *pounds per square inch*) an ein Ende der Kapillare angelegt wird.

10. Trennsubstrat zur Trennung unmodifizierter und modifizierter Mononucleotide und Oligonucleotide, umfassend mindestens 12% Polymer, das nicht mehr als 1% quervernetzt ist in mindestens 50% organischem Lösungsmittel (Volumen:Volumen), wobei das organische Lösungsmittel ein denaturierendes Mittel ist.

11. Trennsubstrat nach Anspruch 10, worin das Polymer ausgewählt ist aus der Gruppe bestehend aus Polyacrylamid, Methylzellulose und Polyvinylalkohol.

12. Trennsubstrat nach Anspruch 11, umfassend ein lineares Polyacrylamid.

13. Trennsubstrat nach Anspruch 12, umfassend etwa 12% bis 20% T-Polyacrylamid.

14. Trennsubstrat nach Anspruch 10, worin das denaturierende Mittel ausgewählt ist aus der Gruppe bestehend aus Formamid, Harnstoff und Natriumdodecylsulfat.

15. Das Trennsubstrat nach Anspruch 14, worin das denaturierende Mittel Formamid ist.

16. Das Trennsubstrat nach Anspruch 15, umfassend etwa 60% Formamid.

17. Das Trennsubstrat nach Anspruch 15 oder 16, ferner Harnstoff umfassend.

## Revendications

1. Procédé de séparation de mononucléotides et d'oligonucléotides non modifiés et modifiés comprenant les étapes consistant à :
(a) fournir un substrat de séparation dans un capillaire haute performance, le substrat de séparation comprenant au moins 12 % de polymère qui n'est pas réticulé à plus de 1 %, dans au moins 50 % (volume:volume) d'un solvant organique, le solvant organique étant un agent de dénaturation;
(b) mettre en contact le substrat de séparation avec les mononucléotides et oligonucléotides à séparer;
(c) appliquer un champ électrique supérieur à 200 volts/cm à travers le substrat de séparation dans le capillaire; et
(d) détecter les mononucléotides et/ou les oligonucléotides séparés.

2. Procédé selon la revendication 1, dans lequel, à l'étape (a), on utilise un substrat de séparation qui comprend un polymère choisi dans le groupe constitué par le polyacrylamide, la méthylcellulose et l'alcool polyvinylique.

3. Procédé selon la revendication 1, dans lequel, à l'étape (a), on utilise un substrat de séparation qui contient de 12 % à 20 % environ de polyacrylamide T.

4. Procédé selon la revendication 3, dans lequel, à l'étape (a), on utilise un substrat de séparation qui contient un gradient linéaire d'environ 13 % à 18 % de polyacrylamide.

5. Procédé selon l'une quelconque des revendications 1 à 4, dans lequel, à l'étape (a), on utilise un dénaturant choisi dans le groupe constitué par le formamide, le dodécylsulfate de sodium et l'urée.

6. Procédé selon la revendication 5, dans lequel, à l'étape (a), on utilise un substrat de séparation qui contient du formamide et éventuellement de l'urée.

7. Procédé selon l'une quelconque des revendications 1 à 6, dans lequel, à l'étape (c), on applique un champ électrique d'environ 400 volts/cm à travers le substrat de séparation dans le capillaire.

8. Procédé selon la revendication 1, comprenant en outre l'étape supplémentaire (e) consistant à éliminer le substrat de séparation du capillaire après l'étape (d).

9. Procédé selon la revendication 8, dans lequel, à l'étape (e), on applique une pression d'au moins 2,11 kg/cm² (environ 30 livres par pouce carré) à une extrémité du capillaire.

10. Substrat de séparation pour la séparation de mononucléotides et d'oligonucléotides non modifiés et modifiés, comprenant au moins 12 % de polymère qui n'est pas réticulé à plus de 1 %, dans au moins 50 % (volume:volume) d'un solvant organique, le solvant organique étant un agent de dénaturation.

11. Substrat de séparation selon la revendication 10, dans lequel le polymère est choisi dans le groupe constitué du polyacrylamide, de la méthylcellulose et de l'alcool polyvinylique.

12. Substrat de séparation selon la revendication 11 comprenant un polyacrylamide linéaire.

13. Substrat de séparation selon la revendication 12, comprenant environ 12 à 20 % de polyacrylamide T.

14. Substrat de séparation selon la revendication 10, dans lequel le dénaturant est choisi dans le groupe constitué par le formamide, l'urée et le dodécylsulfate de sodium.

15. Substrat de séparation selon la revendication 14, dans lequel le dénaturant est le formamide.

16. Substrat de séparation selon la revendication 15, comprenant environ 60 % de formamide.

17. Substrat de séparation selon la revendication 15 ou 16, comprenant en outre de l'urée.
